# EUROPEAN PATENT SPECIFICATION

(11) **EP 1 776 081 B1**
(45) Date of publication and mention of the grant of the patent: **10.08.2011**
(21) Application number: 05756291.0
(22) Date of filing: 03.06.2005
(51) Int. Cl.: A61Q 5/12, A61K 8/00, A61K 8/34, A61K 8/33, A61K 8/04, A61K 8/894

(54) **AQUEOUS COSMETIC COMPOSITIONS**
WÄSSRIGE KOSMETISCHE ZUSAMMENSETZUNGEN
COMPOSITIONS COSMETIQUES AQUEUSES

(30) Priority: 17.06.2004 GB 0413582
(43) Date of publication of application: 25.04.2007
(73) Proprietor: Unilever PLC, London EC4Y 0DY (GB); Unilever N.V., 3013 AL Rotterdam (NL)
(72) Inventor: COXON, Andrew, Charles Unilever R & D Port Sunl.;, Bebington Merseyside CH63 3JW (GB); KUTAY, Susan, Michelle, Port Moody British Columbia V3H 5K5 (CA)
(74) Representative: Whaley, Christopher
(86) International application number: PCT/EP2005/006076
(87) International publication number: WO 2005/123026

(56) References cited:
- WO-A-00/30691
- US-A- 4 874 604
- US-A- 5 221 531
- US-B2- 6 653 353

## Description

### Field of Invention

This invention relates to aqueous cosmetic compositions and to sprays generated therefrom.

### Background

A large number of cosmetic compositions suitable for topical application to the human body have been devised, as have a number of modes of application of such compositions. Traditionally, many cosmetic compositions have comprised high levels of ethanol and/or hydrocarbon propellant. However, as concerns over the effects of such volatile organic carbons (VOCs) upon to environment increase, so does the desire to use compositions comprising less of such components. Water is an excellent, environmentally acceptable alternative component; however, cosmetic compositions comprising water tend to have a wet feel upon application to the human body. This is a serious sensory negative and the present invention provides a means of minimising this problem.

A key feature of the present invention is the presence of a polyalkyleneoxide polysiloxane surfactant. Such surfactants have previously been used in compositions for agricultural use (US 5,558,806 [1996] and WO 97/23281). They have also been used in detergent compositions (US 6,164,296 [2000]; WO 01/00760; EP 786,515 [1997] ; US 2002/0011584; US 6,090,765; WO 01/23518 and WO 02/08373).

Polyalkyleneoxide polysiloxane surfactants have been used in cosmetic compositions described as "odour-absorbing compositions" (WO 98/56339 and WO 98/56341). It is stated that these compositions can be delivered as a liquid via a spray dispenser, a manually operated spray dispenser being preferred.

Polyalkyleneoxide polysiloxane surfactants have also been used in cosmetic compositions suitable for cleaning and/or washing the skin or keratin fibres (US 6,074,633 [2000]).

Polyalkyleneoxide polysiloxane surfactants have also been used in odour-absorbing compositions for use on inanimate surfaces (WO 00/30691; WO 99/55814; and WO 99/55952) and in clear gels suitable for use in personal care products (US 5,623,017 [1997]). Particular polyalkyleneoxide polysiloxane surfactants have also been described as wetting agents in aqueous systems (US 5,401,871 [1995 ] ).

Hairspray with improved adhesion/removability upon washing is described in US 4874604. The homogeneous phase comprises ethanol, a polyalkylenoxide polysiloxane and a cosmetic active but is silent about dimethylether (DME).

US 665353 describes cosmetic or personal care compositions comprising a hair styling polymer having one or more groups selected from acidic functional groups, anionic groups derived from the acidic functional groups or a mixture of said groups, ethanol, DME.

The international publication WO00/30691 relates to a stable, aqueous odor-absorbing composition, preferably for use on inanimate surfaces. The composition comprises from about 0.1 % to about 0.5 %, by weight of the composition, of an emulsion or dispersion comprising long lasting hydrophobic perfume to improve acceptance

The document US5221531 teaches a polymer hair fixative comprising vinyl caprolactam, vinyl pyrrolidone, dimethylaminoethyl methacrylate and acrylate acid, in the form of sodium acrylate, an aqueous-based solution process for making such polymers, and water-based hair spray compositions including such polymer fixatives.

### Summary of Invention

It is an object of the present invention to provide a cosmetic composition, without the use of high levels of VOCs.

In the context of this invention, the meaning of VOC is as defined in Directive 2001/81/EC and UK SI 2002 No. 3118: "all organic compounds arising from human activities, other than methane, which are capable of producing photochemical oxidants by reactions with nitrogen oxides in the presence of sunlight".

It is a further object of the present invention to provide an aqueous cosmetic composition, suitable for spray application to the surface of the human body.

It is a further object of the present invention to provide an aqueous cosmetic composition, suitable for application to the surface of the human body, which has good sensory properties.

A particular object of the present invention is to provide an aqueous cosmetic composition having a reduced wet feel upon application to the surface of the human body.

A further particular object of the present invention is to provide an aqueous cosmetic composition having a reduced drying time once applied to the surface of the human body.

A further particular object of the present invention is to provide an aqueous cosmetic composition, suitable for application to the surface of the human body, which has good sensory properties and delivers a deodorancy benefit.

According to a first aspect of the present invention, there is provided a single phase aqueous cosmetic composition comprising ethanol, dimethylether, a polyalkyleneoxide polysiloxane surfactant, and a cosmetic active.

According to a second aspect of the present invention, there is provided an aqueous cosmetic spray according to the first aspect of the invention.

According to a third aspect of the present invention, there is provided a consumer product comprising an aqueous cosmetic composition according to the first aspect of the invention and a device for producing a spray therefrom.

According to a fourth aspect of the present invention, there is provided a method of cosmetic treatment comprising the spraying of a composition according the first aspect of the invention onto the surface of the human skin.

According to a fifth aspect of the present invention, there is provided the use of a composition according to the first aspect of the invention, or of a spray according to the second aspect of the invention, for giving good sensory properties when used as a cosmetic treatment for the surface of the human body.

The aqueous cosmetic compositions and sprays according to invention preferably have a VOC content of preferably 60% or less, and most preferably 50% or less by weight of the total composition/spray.

### Detailed Description

The present invention involves an aqueous cosmetic composition having good sensory properties when applied, in particular when sprayed, onto the surface of the human body. On application, the composition has a reduced wet feel compared with aqueous cosmetic compositions of the prior art. In addition, the composition may have a reduced drying time, once applied. Without wishing to be bound by theory, it is believed that the benefits of the present invention accrue from the good spreading properties of the composition on the surface of the human body, i.e. on the skin.

Throughout this specification, references to the surface of the human body should be understood as a referring to the skin, as opposed to hair present on the surface of the human body or internal surfaces, such as those present in the oral cavity.

Throughout this specification, percentages should be taken to be by weight, unless otherwise indicated.

An aqueous cosmetic composition/spray is a liquid cosmetic composition/spray that comprises water, in particular, water at 10% or greater of the total composition/spray.
Typically, water is present at from 10 to 90%, in particular at from 20 to 70%. Preferred compositions/sprays comprise water as the major component, excepting any volatile propellant that may also be present; particularly preferred compositions/sprays comprise water as the major component; especially preferred compositions/sprays comprise water at a level of greater than 50% of the composition/spray, excepting any volatile propellant that may also be present.

Throughout this specification, the weight of the total composition/spray should be taken to include all components, including any volatile propellant that may be present in the composition from which the spray is generated, unless otherwise stated. The weight of the "total spray" should be considered to be the same as that of the "total composition" from which it is formed; loss of any volatile propellant during spray formation should not be considered.

The aqueous cosmetic composition is a single phase such as a homogeneous solution.

For reasons of maintenance of good sensory performance upon storage, the pH of the composition/spray according to the invention is preferably from 6 to 8 and more preferably from 6 to 7.5. pH levels outside of the preferred ranges tend to lead to reduced performance due to hydrolysis of the polyalkyleneoxide polysiloxane surfactant.

A spray according to the invention may be distinguished from a composition according to the invention by the fact that the former is a dispersion of droplets of a composition in a gas - the gas typically comprising atmospheric air. A composition according to the invention may be dispersed to form a spray according to the invention by any of the means known in the art (*vide infra*).

It is preferred for sprays according to the invention to have a droplet size distribution such that there are few large droplets, as these can cause poor sensory properties, in particular, a wet feel and slow drying upon application to the surface of the human body. It is also preferred that there are few very small droplets, as these can sometimes cause a hazard with regard to inhalation, when used in proximity to the nose and mouth. For these reasons, a spray according to the invention typically has at least 50%, preferably at least 60%, and more preferably at least 70% of the droplets, by volume, of diameter from 10 microns to 60 microns. For similar reasons, the mean droplet size (D[4,3]) is preferably from 10 to 40 microns.

Droplet size measurements of sprays according to the invention are made 15 cm from the spray orifice, typically using light scattering techniques, typically with instruments such as the Malvern Mastersizer.

The monohydric alcohol ethanol that must be present may be present as an anti-microbial agent or for any other purpose. The monohydric alcohol is ethanol and serves to aid the enhanced sensory benefits offered by the compositions/sprays of the invention, in particular the reduced wet feel and reduced drying time upon application to the surface of the human body. Without wishing to be bound by theory, it is believed that the monohydric alcohol functions synergistically with the polyalkyleneoxide polysiloxane (*vide infra*) to enhance the spreading of the composition upon the surface of the human body. It is also believed that the monohydric alcohol helps improve atomisation, enabling the attainment of the preferred droplet size distributions.

The monohydric alcohol is preferably present at a level of at least 1%, more preferably at a level of at least 5%, and most preferably at a level of at least 10% of the composition/spray. In order that the VOC content of the composition/spray does not rise too high, it is preferred the monohydric alcohol is less than 50%, in particular less than 25%, and especially less than 15% of the total composition/spray, these preferences being particularly relevant when a volatile propellant is also included in the composition/spray.

The polyalkyleneoxide polysiloxane surfactant is believed to aid the good sensory properties, in particular the reduced wet feel reduced drying time, by enhancing the spreading of the composition upon the surface of the human body. Preferred polyalkyleneoxide polysiloxane surfactants are polyalkyleneoxide-modified polydimethylsiloxanes. These latter materials may be of general formula I: 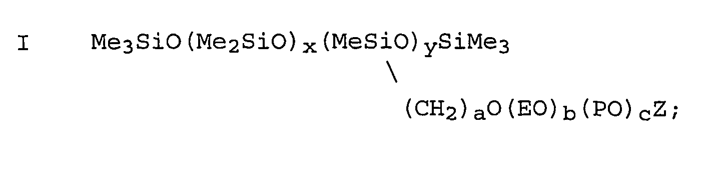 where Me represents methyl; EO represents ethyleneoxy; PO represents 1,2-propyleneoxy; , x is from 0 to 10, preferably from 0 to 5, and most preferably 0; y is from 1 to 8, preferably from 1 to 3, and most preferably 1; a is from 2 to 4, preferably 3; b is from 1 to 15, preferably from 5 to 15; c is from 0 to 14, preferably from 0 to 3; b + c is from 5 to 15, preferably from 6 to 10; and Z can be either hydrogen or a lower alkyl group.

For optimum performance, preferred polyalkyleneoxide polysiloxane surfactants have a molecular weight (MW) of less than 1000, in particular less than 800, and especially less than 700. In polyalkyleneoxide polysiloxane surfactants of formula I, the number of EO units must be sufficient to render the surfactant dispersible or soluble in the composition/spray. When c is non-zero in formula I, the EO and PO units may be distributed randomly, in respective blocks of EO and PO units, or in a combination of random and block distributions.

Preferred polyalkyleneoxide polysiloxane surfactants have an estimated HLB value of from 5-8, the HLB value of the surfactant being estimated from its aqueous solubility and cloud point using the method described by Griffin (Off. Dig. Paint and Varnish Productions Clubs, 28, 446, 1956) and later by Schott (J. Pharm. Sci., 58, 1442, 1969).

Suitable polyalkyleneoxide polysiloxane surfactants that are commercially available may be selected from Silwet L-7280, Silwet L-77, and Silwet L-7608, all available from OSi, and DC Q2-5211, available from Dow Corning Corp.

The polyalkyleneoxide polysiloxane surfactant is typically present at a level from 0.01 to 5%, particularly at from 0.05 to 2.5%, and especially at from 0.05 to 1% by weight of the total composition/spray. When the composition/spray comprises fragrance at from 1% by weight, the polyalkyleneoxide polysiloxane surfactant is preferably present at a level of 0.3% by weight or greater of the total composition/spray.

The cosmetic active that is an essential feature of the invention may be selected from any of those known in the art. Particular classes of active include deodorants; skin benefit agents, including sunscreens, desquamating agents, emollients, humectants, anti-ageing agents, wrinkle-reducing agents, skin whitening agents, sebum reducing agents, and skin moisturisers; hair growth modifiers or eliminators (i.e. depilatories); astringents; cooling agents; and colourants. Deodorant actives, including fragrances and antiperspirant actives, and skin benefit agents, in particular skin moisturisers, are especially suitable for use with the present invention. More than one class of cosmetic active may be used in a composition/spray according to the invention.

The total content of cosmetic active, other than the mentioned monohydric alcohol, may be from 0.1 to 50%; preferably the level is from 0.5 to 25% and most preferably from 1 to 10% of the total composition/spray.

It is highly preferred that the cosmetic active is soluble in water, meaning that it has a solubility in water of 10 g.dm⁻³ or greater at standard temperature and pressure.

Cosmetic compositions comprising a deodorant active are a particular embodiment of the present invention. The deodorant active may be a fragrance or it may be a C1-C4 monohydric alcohol.

Preferred compositions/sprays according to the invention comprise a fragrance. The term 'fragrance' should be considered synonymous with 'perfume' and includes all components of a multi-component fragrance when such is present. The fragrance may comprise simple volatile odoriferous molecules and/or may comprise so-called "deo-perfume" molecules that have anti-microbial properties. Typically, a perfume or fragrance is incorporated at a level of from 0.1 to 5%, in particular 0.2 to 3%, and especially 0.5 to 2% by weight of the total composition/spray.

In preferred embodiments, a deodorant active other than fragrance or a C1-C4 monohydric alcohol is present. When present, a deodorant active other than fragrance or a C1-C4 monohydric alcohol may serve to enhance the deodorancy benefit obtainable from the composition/spray. It may work by absorbing malodour or by preventing malodour formation. Actives that function by preventing the formation of malodour are particularly preferred. (In this description, "preventing" includes partially preventing, i.e. reducing). Such actives may function by preventing the formation of sweat (i.e. as antiperspirants) and/or by preventing the microbial breakdown of sweat components into odiferous molecules. Preferred deodorant actives are anti-microbial agents and function by this latter mechanism.

It is highly preferred that compositions according to the invention do not comprise antiperspirant salts comprising Al(III) or zirconium salts, since such salts are acidic in nature and tend to take the formulation outside of its preferred pH range (*vide supra*).

Preferred anti-microbial agents other than C1-C4 monohydric alcohols are organic anti-microbial agents, in particular agents that are bacteriostats and/or bacteriocides. Typical actives include bacteriostats and/or bacteriocides that are: quaternary ammonium compounds, such as cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). Preferred anti-microbial agents are polyhexamethylene biguanide salts (also known as polyaminopropyl biguanide salts), for example Cosmocil CQ ® available from Arch Chemicals Inc.; poly(alkylene-guanidine) salts or poly(oxyalkylene-guanidine) salts, for example poly(hexamethylene-guanidine hydrochloride) (Akacid^{®}) or poly(triethylene-glycol-guanidine hydrochloride) (Akacid^{®forte}) available from PoC Polymer Produktions GmbH; 2',4,4'-trichloro,2-hydroxy-diphenyl ether (triclosan); and 3,7,11-trimethyldodeca-2,6,10-trienol (farnesol).

The most preferred organic anti-microbial agents are bacteriostats, such as iron(III) chelators, in particular those having an iron(III)-binding coefficient of 10²⁶ or greater. Preferred iron (III) chelators are aminopolycarboxylic acids and salts thereof, in particular the following acids and their salts: diethylenetriaminepentaacetic acid (DTPA), triethylenetetraaminehexaacetic acid (TTHA), and N,N'-ethylenebis[2-(2-hydroxyphenyl)glycine] (EDDHA).

Iron(III) chelators are preferably used in combination with an organic anti-microbial agent that is a phenolic or enolic compound as described in the following paragraph.

The composition/spray may comprise an organic anti-microbial agent that is a phenolic or enolic compound that is (a) a transferrin dissociation promoter that operates by aiding the reduction of iron(III) bound to transferrin to iron(II) and/or (b) an anti-oxidant comprising a tert-butylphenol group. Such materials are described in WO 02/30383. Preferably, such materials are selected from ascorbic acid (and salts thereof), ascorbyl-6-palmitate, butylated hydroxytoluene, 2,2'-ethylidenebis(4,6-di- *tert-*butylphenol), or pentaerythritol tetrakis(3-(3,5-di-tert-butyl-4-hydroxyphenyl)propionate). Butylated hydroxytoluene is especially preferred.

The total amount of organic anti-microbial agent, other than C1-C4 monohydric alcohol, is preferably from 0.1 to 10%, more preferably from 0.1 to 5%, and most preferably from 0.2 to 2% of the of the total composition/spray.

In order to enhance the sensory benefits of compositions/sprays according to the invention, in particular those comprising a deodorant active, a humectant, such a polyol, may be employed. Preferred humectants are glycerol, propylene glycol, and dipropylene glycol; glycerol is particularly preferred.

The sensory benefits of compositions/sprays according to the invention, in particular those comprising a deodorant active, may be enhanced by the presence of an emollient. Such materials are typically oils and are emulsified into an aqueous continuous phase. Suitable oils include silicone oils, such as DC244^{®}, DC245^{®}, DC344^{®} and DC345^{®}, *ex* Dow Corning; alkyl esters, such as PurSyn Ester 2E7^{®} (neopentylglycol dihexanoate), *ex* Exxon-Mobil; benzoate esters such as Finsolv TN (Trade Mark), *ex* Finetex Inc.; hydrogenated polyalkenes, such as Panalane^{®}, *ex* Amoco and PureSyn PAO 2^{®} (hydrogenated polydecene), *ex* Exxon-Mobil; PPG ethers, such as Fluid AP^{®} (PPG-14 butylether), *ex* Union Carbide; isopropyl palmitate; phenylsilicone; and isopropyl myristate.

It should be noted that whilst the presence of an emollient in compositions/sprays according to the invention may enhance sensory benefits in certain respects, they may also detract from the reduced drying time and/or wetness perception benefit. For this reason, it is preferred that an emollient is used at a level below that which causes an increase in drying time and/or wetness perception of a composition according to the invention. In order to determine the drying time and/or wetness perception, the tests described in the Examples may be employed; the emollient-containing composition being compared with a 'control' composition having the emollient replaced by water. Typically, the level of emollient that may be used is from 0.1% to 5%, in particular from 0.1% to 3.5%, and especially from 0.1% to 2% by weight of the total composition. It is preferred that the level of emollient is less than 5 times by weight the level of polyalkyleneoxide polysiloxane surfactant present.

The composition/spray comprises the volatile propellant dimethyl ether (DME).

Typically, the volatile propellant exists as a liquid in compositions according to the invention, being kept in that state by elevated pressure. Preferred volatile propellants, when liquefied by elevated pressure, are miscible with water, typically having a solubility therein of 1.5% or greater by weight at 100 psig and 21°C. Preferably the composition comprises a homogeneous solution of liquefied propellant, water, the polyalkyleneoxide polysiloxane surfactant, the cosmetic active, and, optionally, a nonaqueous cosmetic carrier fluid.

DME is a particularly preferred volatile propellant, having good miscibility with water when liquefied by elevated pressure.

Aqueous compositions according to the invention in which the benefit delivered by the polyalkyleneoxide polysiloxane surfactant is particularly significant comprise DME and ethanol. Such formulations are typically single phase and preferably comprise ethanol, DME, and water in proportions of from 11% to 24% by weight ethanol, from 33% to 55% by weight DME, and from 27% to 50% by weight water based upon the total weight of ethanol, DME, and water present in the composition. It is further preferred that the weight ratio of DME to ethanol is from 2:1 to 3.5:1. At these preferred amounts and/or further preferred ratios, the polyalkyleneoxide polysiloxane surfactant delivers its most significant benefits.

In order to minimise VOC content, it is preferred that the compositions/sprays of the present invention do not comprise more volatile propellant than is necessary. It is preferred that volatile propellant is used at a level of 60% or less, more preferably 50% or less and most preferably 40% or less by weight of the total composition/spray. For functional reasons, however, it may be necessary to use a volatile propellant at 25% or greater, 30% or greater, or even 35% or greater by weight of the total composition/spray.

In order to minimise VOC content, it is preferred that the total content of volatile propellant and monohydric alcohol is equal to 60% or less, and most preferably 50% or less, of the total composition/spray.

Other components that may be present include additional emulsifiers or wash-off agents; these are generally nonionic surfactants such as fatty alcohol ethoxylates or PEG esters of fatty acids; inorganic electrolytes, such as sodium chloride or sodium sulphate; rheology modifiers, such as hydroxypropyl celluloses; silicone gum, such as Q2 1501, *ex* Dow Corning; colourants; and preservatives, such as Glydant or parabens.

The spraying device used with the compositions of the invention may be one of two possible types. In one embodiment, the device comprises a means for mechanically pressurising the composition. Typical of such devices are those utilising manual pressurisation of the composition, such as those known as 'squeeze spray' and 'trigger spray' devices.

In a second embodiment, used with compositions comprising volatile propellant, the spraying device comprises a pressure canister and a valve. In such embodiments, the volatile propellant contributes to the dispersion of the composition into a spray.

Manually activated spraying dispensers are generally not capable of achieving the preferred droplet size distributions for sprays according to the invention (vide supra) and, for this reason, they are not preferred. It is preferred that spray generation involves the use of compositions comprising a volatile propellant and a spray device comprising a pressure canister and a valve. It is particularly preferred that the spray device comprises a swirl chamber nozzle, as known in the art, for enhancing the spray quality and attaining the preferred droplet size distributions for sprays according to the invention.

### Examples

In the following examples, comparative examples are indicated by letters and examples according to the invention are indicated by numbers. All amounts are percentages by weight of the total composition.

The compositions indicated in Table 1 were prepared by methods known in the art. -

**Table 1: Aerosol Compositions**

| **Example** | **A** | **1** | **2** |
|---|---|---|---|
| Silwet L-7608¹ | - | 0.05 | - |
| Silwet L7280² | - | - | 0.05 |
| Cremophor CO60³ | 0.05 | - | - |
| Denatured ethanol | 12.50 | 12.50 | 12.50 |
| DME⁴ | 37.50 | 37.50 | 37.50 |
| Water | To 100 | To 100 | To 100 |
| Speading⁵ (mm) | 8.5 | 23.5 | 24.0 |
| Drying time⁶ (s) (at dose(g)) | 115 (0.87) | - | 79 (0.91) |
| | 119 (1.07) | 62 (1.05) | - |
| | 142 (1.25) | 73 (1.19) | - |
| | 171 (1.41) | 120 (1.48) | - |

| | | | |
|---|---|---|---|
| 1. Poly(ethyleneoxide)-modified heptamethyltrisiloxane; MW: 600; *ex* Witco. According to general formula I, with x = 0, y = 1, a = 3, and c = 0. 2. Poly(ethyleneoxide/propyleneoxide)-modified heptamethyltrisiloxane, MW: 600; *ex* Witco. According to general formula I, with x = 0, y = 1, a = 3, and b:c = 3:2. 3. PEG-60 hydrogenated castor oil, *ex* BASF. 4. Dimethyl ether (propellant). 5. Average of 4 repeat measurements made on base compositions lacking propellant (details below). 6. As judged by panellists (details below). In each case, the Example according to invention was judged to dry significantly faster (at 98% significance or greater) than the Control Example on the one on the same line. | | | |

For the spreading measurements, base compositions were dripped, from a height of 5 mm, onto a sheet of Epson Ink Jet Transparancy film (S041063) which acted as a model for the skin surface. The individual drops (volume 40 :1) were allowed 1 minute to spread, after which time the diameters reached were measured. The results indicate much greater spreading for the compositions/sprays of the invention and the inventors believe that this correlates with reduced wetness perception and increased speed of drying (*vide infra*).

The drying times indicated were judged by a trained sensory panel. The products were tested in pairs, the compared products/doses being those on the same line. Using a conventional spray device comprising a pressure canister, a valve and a swirl chamber nozzle, a test co-ordinator sprayed a first product to a first volar forearm of a panellist and the panellist was then asked to record the time for the product to dry. This was repeated with the second product on the panellist's other volar forearm. A balanced design was used to eliminate any left-right bias. Droplet size measurements on the sprays indicated that greater than 70% of the droplets, by volume, were from 10 microns to 60 microns in diameter, in each case.

The drying time results illustrate the much faster drying of the compositions/sprays according to the invention.

**Table 2: Further Aerosol Compositions**

| **Example** | **C** | **3** | **D** | **4** | **E** | **5** |
|---|---|---|---|---|---|---|
| Silwet L-7608¹ | - | 0.50 | - | 0.50 | - | 0.50 |
| Cremophor CO60¹ | 0.05 | - | 0.05 | - | 0.05 | - |
| Denat. ethanol | 12.50 | 12.50 | 12.50 | 12.50 | 20.00 | 20.00 |
| DME¹ | 37.50 | 37.50 | 37.50 | 37.50 | 50.00 | 50.00 |
| DTPA² | 0.50 | 0.50 | 0.50 | 0.50 | 0.50 | 0.50 |
| AMP 95³ | 0.40 | 0.40 | 0.37 | 0.37 | 0.37 | 0.37 |
| BHT⁴ | 0.10 | 0.10 | 0.10 | 0.10 | 0.10 | 0.10 |
| Fragrance | 1.50 | 1.50 | 1.50 | 1.50 | 1.50 | 1.50 |
| Water | to 100 | to 100 | to 100 | to 100 | to 100 | to 100 |
| Speading¹ (mm) | 10.5 | 22.0 | 10.5 | 21.8 | 13.3 | 18 |
| Drying time¹ (s) | 85 | 63 | 81 | 65 | - | - |
| (at dose (g)) | (0.99) | (0.94) | (0.93) | (0.91) | - | - |
| Wetness score⁵ : | | | | | | |
| Underarm, 1 min. | - | - | 24 | 19 | - | - |
| Forearm, 1 min. | - | - | 34 | 27 | - | - |
| Forearm, 2 min. | - | - | 22 | 16 | 15 | 11 |

| | | | | | | |
|---|---|---|---|---|---|---|
| 1. As described beneath Table 1. 2. Diethylenetriaminepentaacetic acid. 3. 2-amino-2-methyl-1-propanol, 95% in water, *ex* Angus Chemical. 4. Butylated hyroxytoluene. 5. As judged by panellists (details below). In each case, the Example according to invention was perceived to be significantly less wet (at a significance of greater than 95%) than the Control Example on the one on the same line. | | | | | | |

The compositions indicated in Table 2 were prepared by methods known in the art.

The spreading measurements and drying times were obtained by the method as described for the compositions of Table 1, *mutatis mutandis.* The results indicate much greater spreading and increased speed of drying for the compositions/sprays of the invention.

The wetness scores were obtained using a panel of trained assessors. The panellists were asked to apply the products to freshly washed and dried volar forearms and underarms. After the indicated times, the panellists were asked to assess the wetness using a simple 0 to 100 line scale with anchor points of 'not wet' at 0 and 'very wet' at 100. The results indicate a much lower perception of wetness for the compositions/sprays according to the invention and confirm the correlation between enhanced spreading and reduced wetness perception.

## Claims

1. A single phase aqueous cosmetic composition comprising ethanol, dimethylether, a polyalkyleneoxide polysiloxane surfactant, and a cosmetic active.

2. An aqueous cosmetic composition according to claim 1, comprising a fragrance.

3. An aqueous cosmetic composition according to claim 1 or 2, comprising a deodorant active other than ethanol or fragrance.

4. An aqueous cosmetic composition according to any of preceding claims, having a VOC content of 60% or less by weight.

5. An aqueous cosmetic composition according to claim 1, comprising ethanol, DME, and water in proportions of from 11% to 24% by weight ethanol, from 33% to 55% by weight DME, and from 27% to 50% by weight water based upon the total weight of ethanol, DME, and water present in the composition.

6. An aqueous cosmetic composition according to claim 5, wherein the weight ratio of DME to ethanol is from 2:1 to 3.5:1.

7. A method of cosmetic treatment comprising the spraying of a composition according to any of the preceding claims onto the surface of the human skin.

8. The use of a composition according to any of claims 1 to 6, for giving good sensory properties when used as a cosmetic treatment for the surface of the human body.

9. The use of a composition according to any of claims 1 to 11, for giving a reduced wet feel when used as a cosmetic treatment for the surface of the human body.

## Patentansprüche

1. Einphasige wässrige kosmetische Zusammensetzung, die Ethanol, Dimethylether, ein Polyalkylenoxidpolysiloxan-Surfactant und einen kosmetischen Wirkstoff umfasst.

2. Wässrige kosmetische Zusammensetzung gemäß Anspruch 1, die einen Duftstoff umfasst.

3. Wässrige kosmetische Zusammensetzung gemäß Anspruch 1 oder 2, die einen anderen desodorierenden Wirkstoff als Ethanol oder Duftstoff umfasst.

4. Wässrige kosmetische Zusammensetzung gemäß einem der vorangehenden Ansprüche, die einen VOC-Gehalt von 60 Gewichts-% oder weniger hat.

5. Wässrige kosmetische Zusammensetzung gemäß Anspruch 1, die Ethanol, DME und Wasser in Anteilen von 11 Gewichts-% bis 24 Gewichts-% Ethanol, 33 Gewichts-% bis 55 Gewichts-% DME und 27 Gewichts-% bis 50 Gewichts-% Wasser, bezogen auf das Gesamtgewicht von Ethanol, DME und Wasser, die in der Zusammensetzung vorhanden sind, umfasst.

6. Wässrige kosmetische Zusammensetzung gemäß Anspruch 5, wobei das Gewichtsverhältnis von DME zu Ethanol 2:1 bis 3,5:1 ist.

7. Verfahren zur kosmetischen Behandlung, das Sprühen einer Zusammensetzung gemäß einem der vorangehenden Ansprüche auf die Oberfläche der menschlichen Haut umfasst.

8. Verwendung einer Zusammensetzung gemäß einem der Ansprüche 1 bis 6 zur Verleihung von guten sensorischen Eigenschaften, wenn sie als kosmetische Behandlung für die Oberfläche des menschlichen Körpers verwendet wird.

9. Verwendung einer Zusammensetzung gemäß einem der Ansprüche 1 bis 11 zur Verleihung eines verringerten Feuchtegefühls, wenn sie als kosmetische Behandlung für die Oberfläche des menschlichen Körpers verwendet wird.

## Revendications

1. Composition cosmétique aqueuse monophasique comprenant de l'éthanol, de l'éther diméthylique, un tensioactif d'oxyde de polyalkylène-polysiloxane, et une substance cosmétique active.

2. Composition cosmétique aqueuse selon la revendication 1, comprenant une fragrance.

3. Composition cosmétique aqueuse selon les revendications 1 ou 2, comprenant une substance déodorante active autre que l'éthanol ou la fragrance.

4. Composition cosmétique aqueuse selon l'une quelconque des revendications précédentes, ayant une teneur en COV de 60 % en poids ou moins.

5. Composition cosmétique aqueuse selon la revendication 1, comprenant de l'éthanol, du DME, et de l'eau en des proportions de 11 à 24 % en poids d'éthanol, de 33 à 55 % en poids de DME, et de 27 à 50 % en poids d'eau, sur la base du poids total de l'éthanol, du DME et de l'eau présents dans la composition.

6. Composition cosmétique aqueuse selon la revendication 5, dans laquelle le rapport en poids du DME à l'éthanol est de 2:1 à 3,5:1.

7. Méthode de traitement cosmétique comprenant la pulvérisation d'une composition selon l'une quelconque des revendications précédentes sur la surface de la peau humaine.

8. Utilisation d'une composition selon l'une quelconque des revendications 1 à 6, pour conférer de bonnes propriétés sensorielles quand elle est utilisée à titre de traitement cosmétique pour la surface du corps humain.

9. Utilisation d'une composition selon l'une quelconque des revendications 1 à 6, pour conférer une sensation humide réduite quand elle est utilisée à titre de traitement cosmétique pour la surface du corps humain.
